Europäisches Patentamt

European Patent Office

Office européen des brevets

(11) Publication number: **0 459 516 A1**

# EUROPEAN PATENT APPLICATION

(21) Application number: 91108963.9

(22) Date of filing: 31.05.91

(51) Int. Cl.5: **A61K 37/02, A61K 9/48**

(30) Priority: 01.06.90 JP 145231/90
24.05.91

(43) Date of publication of application:
**04.12.91 Bulletin 91/49**

(84) Designated Contracting States:
**DE FR GB IT**

(71) Applicant: **Kirin-Amgen, Inc.**
**1900 Oak Terrace Lane**
**Thousand Oaks California 91320(US)**

(72) Inventor: **Nomura, Hideaki**
**A304 Kirin-Biiru-Kuragano-Shataku, 11**
**Miyahara-cho**
**Takasaki-shi, Gunma-ken(JP)**
Inventor: **Maruyama, Kazutoshi**
**391 Kami-Koide-machi**
**Maebashi-shi, Gunma-ken(JP)**

(74) Representative: **Brown, John David et al**
**FORRESTER & BOEHMERT**
**Widenmayerstrasse 4/I**
**W-8000 München 22(DE)**

(54) **Oral dosage form of biologically active proteins.**

(57) An oral dosage form comprising
(a) granulocyte colony-stimulating factor or erythropoietin,
(b) surfactant(s),
(c) fatty acid(s), and
(d) enteric material.

With the oral drug preparations provided by the present invention, therefore, inactivation of the principal ingredient during the process of pharmaceutical manufacturing has been shown to be avoided along with proven effects such as enhanced absorption of the principal ingredient from the intestinal tract particularly as a result of the addition of fatty acid to the drug composition.

These may be said not only to lead to a dosage reduction but also to facilitate accurate dose control and increase the practical usefulness of the bioactive proteins in the form of oral preparations.

*Fig. I*

EP 0 459 516 A1

## BACKGROUND OF THE INVENTION

[Field of Invention]

The present invention relates to oral dosage form containing the biologically active proteins, granulocyte colony-stimulating factor (G-CSF) and erythropoietin (EPO), as active ingredients.

[Prior Art]

G-CSF is a hematopoietic factors and has been demonstrated to occur in the medium of cultures of human bladder carcinoma cell line 5637 (ATCC HT8-9) (Welte et al.: Proc. Natl. Acad. Sci. U.S.A., 82, 1526~1530 (1985)). The DNA base sequence coding the gene for this hematopoietic factor has been determined (Japanese Patent Application Laying-Out (KOHYO) No. 500636/88), thus making possible the production of G-CSF by the technique of genetic recombination.

G-CSF is effective in the treatment of common hematopoietic disorders, in that of hematopoietic disorders caused by chemotherapy or radiotherapy, and in bone marrow transplantation (Welte et al.: as above).

Pharmaceutical preparations containing G-CSF, supplemented with various substances added to G-CSF to prevent its inactivation and adsorption to the wall of container on storage as well as for stabilization of G-CSF, have been applied for patents, i.e. a preparation with an added surfactant (Japanese Patent Application Laid-Open (KOKAI) No. 146826/88), one with an added saccharide (Japanese Patent Application Laid-Open (KOKAI) No. 146827/88), one with an added high molecular weight compound (Japanese Patent Application Laid-Open (KOKAI) No. 146828/88), one with an added amino acid, sulfurated reducing agent or antioxidant (Japanese Patent Application Laid-Open (KOKAI) No. 146829/88), and one with an added protein (Japanese Patent Application Laid-Open (KOKAI) No. 152326/88), respectively. All these preparations, however, are in the form of injectable solution.

A sustained-release preparation comprising G-CSF incorporated into a vehicle consisting of a biocompatible high polymer to prevent inactivation of G-CSF and to extend the duration of its drug effect, when administered, has also been applied for a patent Japanese Patent Application Laid-Open (KOKAI) No. 91325/88). Furthermore, an oral enteric-coated preparation composed of G-CSF, a surfactant, a substance in a solid state at ordinary temperature and soluble in organic solvents and an enteric material, for oral administration, has been reported (PCT Laid-Open Official Gazette (KOKAI-KOHO) WO90/01329).

Meanwhile, EPO is a hematopoietic hormone consisting of a glycoprotein with an approximate molecular weight of 34,000 largely produced in and secreted by the kidneys, which functions to stimulate the differentiation of erythroblastic precursor cells into erythrocytes.

EPO has been shown to be a substance useful in erythropoietin therapy, represented by therapy for anemia, in man and other mammals.

As an EPO from natural sources, for example, a preparation purified from urine of patients with aplastic anemia is known (Miyake et al.: Journal of Biological Chemistry, vol. 252, No. 15, pp.5558-5564, 1977). The said method, however, provides only a limited supply because of the low yields and therefore is not suitable for industrial, large-scale production. A method for production of EPO with a high productivity utilizing recombinant DNA technique thus has been introduced (Japanese Patent Publication (KOKOKU) No. 17156/90).

As pharmaceutical preparations containing EPO, there have been applied for patents a preparation with serum albumin, dextran or polyethylene glycol added to EPO for its stabilization or prevention of its adsorption to the wall of container (Japanese Patent Application Laid Open (KOKAI) No. 91131/86 and (KOKAI) No. 97229/86) and a preparation for intranasal administration consisting of EPO and an aqueous and/or non-aqueous medium containing a surfactant added for the said purpose ((KOKAI) No. 89627/87).

Biologically active proteins are generally liable to be degraded by gastric acid and by enzymes in the gastrointestinal lumen or wall, and it is extremely difficult to have them absorbed from the digestive tract because of their molecular sizes and complicated molecular structures. Eventually, their clinical application has been limited to parenteral administration by intravenous, subcutaneous or intramuscular injection.

Such parenteral administration by injection, nevertheless, entails problems of inflicting pain on the patient, of causing damage to the tissue at the site of injection, and of not permitting self-medication. To circumvent these problems, various attempts have been made with other methods of closing. These include, for example, intranasal administration (Pharm. Res., 21, 105 (1989)), oral administration (Res. Commun. Pathol. Pharmacol., 63, 53 (1989)), tracheal administration (Diabetes, 20, 552 (1971)), transdermal administration (J. Pharm. Sci., 78, 376 (1989)) and rectal administration (J. Pharm. Pharmacol., 33, 334 (1981)), which all entail problems and none has proven to be of practical use.

The same holds true with G-CSF and EPO as well. Oral preparations are desired, of which the method of administration has for many decades been present and accessible and which is very simple, does not inflict pain on the patient and permits self-medication. However, attempts of oral administration of these substances have been extremely rare as compared with other methods of dosing. Only such dosing methods as, for example, one in which the active ingredient is enclosed in liposomes (Chem. Pharm. Bull., 30, 2245 (1982)), one in which the active ingredient is entrapped in microcapsules (Japanese Patent Application (TOKUGAN) No. 190833/88) and another in which the active ingredient is coated with azoaromatic copolymer (Science, 233, 1081 (1986)) are known.

Thus, an object of the present invention is to provide an oral preparation of G-CSF or EPO with obviation of these knotty points.

SUMMARY OF THE INVENTION

In view of the above-described state of affairs, the present inventors pursued studies aiming at development of pharmaceutical preparations that afford an efficient absorption of G-CSF or EPO from the gastrointestinal tract and at establishment of a method of manufacturing the preparations, as a result, they found that it was effective for these purposes to add surfactant(s), fatty acid(s) and enteric material(s), thus leasing to attainment of this invention.

That is, the oral preparations provided by the present invention is characterized by comprising G-CSF or EPO, surfactant(s), fatty acid(s) and enteric material(s).

BRIEF DESCRIPTION OF DRAWINGS

Fig. 1    graphically shows the result of Experiment 1.
Fig. 2    graphically shows the result of Experiment 2.
Fig. 3    graphically shows the result of Experiment 3.
Fig. 4    graphically shows the result of Experiment 4.

DETAILED DESCRIPTION OF THE INVENTICN

G-CSF isolated and purified from a product yielding from a transformant obtained by transformation via genetic recombination may be used as the G-CSF in the preparations of this invention. Preferably, human G-CSF possessing substantially the below-described amino acid sequence and produced by Escherichia coli via genetic recombination is used. The term "substantially" here denotes that the amino acid sequence either is identical with that of natural human G-CSF or contains one or more amino acid alterations (i.e., deletion, addition, insertion and displacement) which do not bring forth any detrimental functional dissimilarity to natural G-CSF.

(Met)n

Thr Pro Leu Gly Pro Ala Ser Ser Leu Pro Gln

Ser Phe Leu Leu Lys Cys Leu Glu Gln Val Arg

Lys Ile Gln Gly Asp Gly Ala Ala Leu Gln Glu

Lys Leu Cys Ala Thr Tyr Lys Leu Cys His Pro

Glu Glu Leu Val Leu Leu Gly His Ser Leu Gly

Ile Pro Trp Ala Pro Leu Ser Ser Cys Pro Ser

Gln Ala Leu Gln Leu Ala Gly Cys Leu Ser Gln

Leu His Ser Gly Leu Phe Leu Tyr Gln Gly Leu

Leu Gln Ala Leu Glu Gly Ile Ser Pro Glu Leu

Gly Pro Thr Leu Asp Thr Leu Gln Leu Asp Val

Ala Asp Phe Ala Thr Thr Ile Trp Gln Gln Met

Glu Glu Leu Gly Met Ala Pro Ala Leu Gln Pro

Thr Gln Gly Ala Met Pro Ala Phe Ala Ser Ala

Phe Gln Arg Arg Ala Gly Gly Val Leu Val Ala

Ser His Leu Gln Ser Phe Leu Glu Val Ser Tyr

Arg Val leu Arg His Leu Ala Gln Pro

(n=0 or 1)

More preferably, a recombinant human G-CSF with the above amino acid sequence wherein n = 1 is used. The above-described recombinant human G-CSF may be obtained, for example, in accordance with the disclosure of Japanese Patent Application Laying-Out (TOKUHYO) No. 500636/88.

The EPO which may be used in the present invention, on the other hand, means not only those naturally produced in humans and other mammals such as monkeys but also polypeptides, which possesses the biological activity of natural EPO and is obtained by recombinant DNA technique represented by the said recombinant EPO disclosed in the TOKKO No. 17156/90, and also their analogues possessing the biological activity of natural EPO.

The EPO used in this invention is preferably a human type EPO, or, more preferably, a human type EPO obtained by recombinant DNA technique.

Surfactants used in the present invention include anionic surfactants such as sodium laurylsulfate, dioctyl sodium sulfosuccinate and dioctyl sodium sulfonate, cationic surfactants such as benzalkonium chloride and benzethonium chloride, and nonionic surfactants such as lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methylcellulose and carboxymethyl cellulose. One or a mixture of two or more of these surfactants is used. Preferably, these surfactants are added usually in a ratio between 0.1 and 1000 parts by weight to one part by weight of G-CSF. The same holds also for EPO.

Fatty acids used in the present invention include those contained in edible plants such as oleic acid, linoleic acid and linolenic acid, of which one or a mixture of two or more is used. Preferably, these fatty acids are added usually in a ratio between 0.1 and 1000 parts by weight to one part by weight of G-CSF. The same holds good with EPO.

Among these fatty acids, oleic acid and linoleic acid are especially preferable.

Enteric materials used in this invention include cellulose acetate phthalate, methyl acrylate-methacrylate copolymer, carboxymethylcellulose, hydroxypropyl methylcellulose phthalate and hydroxypropyl methylcellulose acetate succinate, of which one or a mixture of two or more is used. The enteric materials may take the form of enteric capsules, enteric coating and the like.

In the present invention, excipients are used when deemed necessary. The excipients used in this invention include starches, sugars, inorganic substances, organic acids, celluloses, synthetic and semisynthetic polymers, amino acids and the like. The starches include corn starch, wheat starch, potato starch and the like. The sugars include lactose, glucose, saccharose, fructose, D-sorbitol, D-mannitol, inositol, sucrose and the like. The inorganic substances include magnesium stearate, calcium phosphate, calcium hydrogen phosphate, precipitated calcium carbonate, sodium hydrogen carbonate, magnesium carbonate, sodium chloride, calcium sulfate and so forth. The organic acids include succinic acid, tartaric acid, citric acid, fumaric acid, maleic acid, malonic acid, glutaric acid, adipic acid, malic acid, gluconic acid, glucuronic acid and the like. The celluloses include microcrystalline cellulose, methylcellulose, ethylcellulose, hydroxyethyl cellulose, hydroxypropyl cellulose, carboxypropyl cellulose, carboxymethyl cellulose sodium and so forth. The synthetic and semisynthetic polymers include polyvinyl alcohol, carboxyvinyl polymer, polyethylene glycol, polyvinylpyrrolidone, sodium polyacrylate and the like. The amino acids include L-arginine, D,L-methionine, L-phenylalanine, L-glutamic acid and so forth.

In the oral preparations provided by the present invention, absorption of the principal ingredient, biologically active protein, from the gastrointestinal tract is enhanced by especially adding fatty acid to the drug composition. This is considered to be due to synergistic actions of fatty acid and surfactant on the gastrointestinal mucosa with a consequent increase in absorbability of the biologically active protein, or due to formation of a complex with increased absorbability through combined actions of fatty acid and surfactant upon the bioactive protein.

The following procedures are considered practicable as the method for manufacturing the oral drug preparations provided by this invention. For example, a surfactant and a fatty acid are dissolved in a buffer solution or distilled water and sonicated. To this solution, a solution of G-CSF or EPO is added and, when necessary, an excipient is also added; the resultant mixture is mixed by stirring at low temperature. The solution obtained is then lyophilized into powder. An excipient is added to the powder if necessary, and, after mixing, the mixture is sieved to an even grain size and filled in enteric capsules. In another method, a surfactant and a fatty acid are dissolved in a buffer solution or distilled water. To the resulting solution, a bulk solution of the biologically active protein is added and, if necessary, an excipient is also added, followed by stirring at low temperature. The solution obtained is then lyophilized into powder. An excipient is added to the powder if necessary, and the resulting mixture is sieved to even grain sizes and formed into tablet. The granules are enteric-coated and filled in hard gelatin capsules.

Alternatively, an excipient is added to the powder if necessary, and the resulting mixture is sieved into granules of even grain sizes.

Organic solvents may be used in manufacturing the oral dosage form provided by the present invention. Preferably, however, a buffer solution or distilled water is used in order to avoid inactivation of the principal ingredient, G-CSF or EPO, as well as to eliminate the influence of residual solvents. Furthermore, it is preferable to obviate thermal treatment in the manufacturing process, as in the above-described manufacturing examples, in order to minimize inactivation of G-CSF or EPO. The lyophilization is not an essential process in the manufacturing of the oral dosage form provided by the present invention but is effective to provide high contents of the principal ingredient in the pharmaceutical preparations.

The following methods are practicable for the manufacturing of the enteric capsules. For example, an enteric material is dissolved in an organic solvent or dispersed in distilled water, and (hard or soft) capsules are immersed in or sprayed with the resulting solution and then allowed to dry. In another method, an enteric material is dissolved in an organic solvent or dispersed in distilled water, and capsule body molds and cap molds are immersed in the resulting solution, followed by drying and removal of formed enteric material capsules from the molds. In another method, an enteric material is dissolved in an organic solvent or dispersed in distilled water, and outer parts of hard gelatin capsule bodies and caps are immersed in the resulting solution and then dried. The dried outer parts were placed in distilled water to eliminate inner gelatin, followed again by drying to obtain enteric capsules.

The following examples will serve as further embodiments to illustrate the present invention.

Example 1

0.20 g of polyoxyethylene hydrogenated castor oil 60 and 0.28 g of linoleic acid were dissolved in 100

5

ml of distilled water and sonicated. To this solution, 200 ml of a G-CSF solution (0.50 mg/ml) were added and the resultant mixture was stirred overnight in a cold room, followed by lyophilization to obtain a homogeneous freeze-dried preparation.

4.0 g of hydroxypropyl methylcellulose phthalate were dissolved in 10 ml of a methanol-methylene chloride mixture (3:10), and outer parts of hard gelatin capsule bodies and caps were then immersed in the resultant solution and, subsequently, thoroughly dried.

The dried outer parts were placed in distilled water to remove inner gelatin, and dried again to obtain enteric capsules. The above-described lyophilized preparation was filled in these enteric capsules. Besides, an enteric capsule preparation of G-CSF was prepared in the same manner as above but without the addition of linoleic acid.

Example 2

0.40 g of polyoxyl 40 stearate and 0.2 g of oleic acid were dissolved in 100 ml of distilled water and sonicated. 200 ml of a G-CSF solution (0.50 mg/ml) and 1.0 g of lactose were added to this solution, and the resultant mixture was stirred overnight in a cold room, followed by lyophilization to obtain a homogeneous freeze-dried preparation. This preparation was made into granules with added 0.10 g of hydroxypropyl cellulose, and filled in enteric capsules which had been prepared in the same manner as in Example 1.

Example 3

0.50 g of polyoxy 40 stearate and 0.20 g of linoleic acid were dissolved in 100 ml of citrate buffer solution and sonicated. To this solution, 100 ml of a G-CSF solution (2.0 mg/ml), 0.10 g of L-arginine and 1.0 g of saccharose were added, and the resulting mixture was stirred overnight in a cold room, followed by freeze-drying to obtain a homogeneous lyophilized preparation. To this preparation, 0.10 g of succinic acid 0.10 g of microcrystalline cellulose and 0.10 g of magnesium stearate were added, mixed, and formed into tablets. The tablets were enteric-coated by spraying with a hydroxypropyl methylcellulose acetate succinate suspension and subsequent drying.

Example 4

0.40 g of sucrose fatty acid ester and 0.10 g of linoleic acid were dissolved in 100 ml of citrate buffer solution and sonicated. To this solution, 200 ml of a G-CSF solution (2.0 mg/ml) and 1.0 g of lactose were added and stirred overnight in a cold room, and the mixture was freeze-dried to obtain a homegeneous lyophilized preparation. This preparation was made into granules with added 0.1 g of hydroxypropyl cellulose. The granules were enteric-coated by spraying with a hydroxypropyl methylcellulose acetate succinate suspension and subsequent drying, and filled in hard gelatin capsules.

Example 5

0.40 g of sucrose fatty acid ester and 0.30 g of linoleic acid were dissolved in 100 ml of distilled water and sonicated. To this solution, 200 ml of a solution of EPO ($25 \times 10^4$ lu/ml) were added, and the mixture was stirred overnight in a cold room and freeze-dried to obtain a homogeneous lyophilized preparation. To this preparation, 0.10 g of sodium phosphate was added, and the resulting mixture was filled in enteric capsules made of hydroxypropyl methylcellulose.

Example 6

A solution of 0.30 g of polyoxyl 40 stearate and 0.20 g of oleic acid in 100 ml of distilled water was prepared and subjected to ultrasonification. To this solution, 200 ml of a solution of EPO ($25 \times 10^4$ lu/ml) and 1.0 g of lactose were added, and the mixture was stirred overnight in a cold room and then freeze-dried to obtain a homogeneous lyophilized preparation. This preparation was sieved to an even grain size, mixed with added 0.20 g of microcrystalline cellulose and 0.05 g of magnesium stearate, and formed into tablets. The tablets were enteric-coated by spraying with a carboxymethylcellulose suspension and subsequent drying.

Example 7

6

0.30 g of polyoxyl 40 stearate are 0.20 g of linoleic acid were dissolved, with stirring, in 100 ml of distilled water and sonicated. To this solution, 200 ml a solution of EPO ($25 \times 10^4$ lu/ml), 1.0 g of D-mannitol and 0.10 g of L-arginine were added, stirred overnight in a refrigerator and freeze-dried to obtain a homogeneous lyophilized preparation. This preparation was made into granules with added 0.10 g of hydroxypropyl cellulose, and the granules were enteric-coated by spraying with a hydroxypropyl methylcellulose acetate succinate suspension and subsequent drying, and filled in hard gelatin capsules.

Example 8

0.80 g of polyoxyl 40 stearate and 0.80 g of linoleic acid were dissolved in 100 ml of citrate buffer solution and sonicated. To 8 ml of this solution, 2 ml of G-CSF solution (73 mg/ml) was added and the resultant mixture was stirred overnight in a cold room to be a test solution.

In addition, three other test solutions were prepared according to the same procedure with the addition of oleic acid, linoleinic acid instead of linoleic acid or without the addition of fatty acid (for control solution).

Example 9

Five solutions containing 1.6 g of polyoxyl 40 stearate and 0 g, 0.4 g, 1.6 g, 3.2 g or 6.4 g of linoleic acid respectively in 100 ml of citrate buffer solution were prepared and sonicated. To 9 ml of each solution, 1 ml of G-CSF solution (20 mg/ml) was added and stirred overnight in a cold room to be a test solution.

Experiment 1

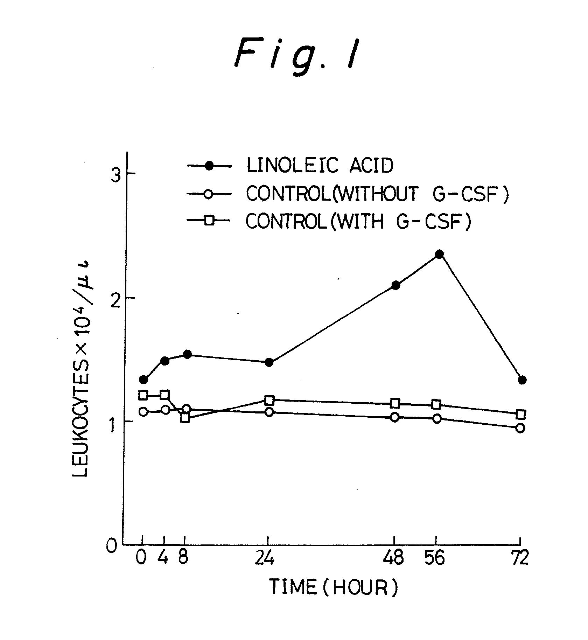

Male beagle dogs (13- to 15-month-old) having been fasted for 20 hours (but allowed free access to water) were dosed orally with a drug dosage form of this invention containing linoleic acid prepared in Example 1 or with the same dosage form but not containing linoleic acid, in a dose of 2.2 mg/kg of G-CSF. Serial 0.5 ml blood samples were taken serially following the administration from the antebrachial vein of each dog. Each blood sample was collected in an EDTA-treated polystyrene tube and subjected to determination of blood total leukocyte count on an automated blood cell counter (Microcell Counter Sysmex CC-180A). Serial total leukocyte counts are shown in Figure 1.

As seen, the total leukocyte count increased from 24 ~ 48 hours after oral administration of the drug dosage form containing linoleic acid provided by this invention, reached a peak at 56 hours post-dosing and returned to the initial level by 72 hours post-dosing. In contrast, no such pharmacological effect was observed in dogs receiving the drug preparation not containing linoleic acid and in those receiving a preparation not containing G-CSF (controls).

Experiment 2

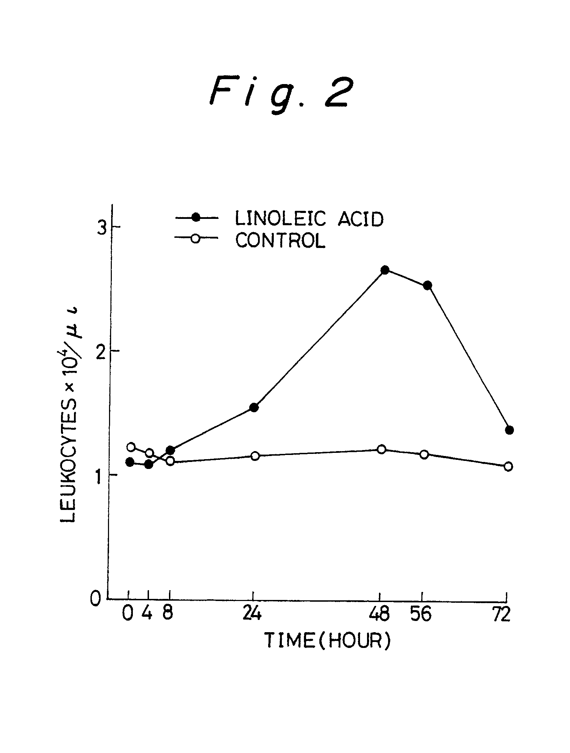

Male beagle dogs (13- to 15-month-old) having been fasted for 20 hours (but allowed free access to water) were dosed orally with a drug dosage form prepared in Example 3 in a dose of 2.0 mg/kg of G-CSF. 0.5 ml blood samples were taken serially following the administration from the antebrachial vein of each dog. Each blood sample was collected in an EDTA-treated polystyrene tube and subjected to determination of blood total leukocyte count on an automated blood cell counter (Microcell Counter Sysmex CC-180A). Serial total leukocyte counts are shown in Figure 2.

As seen, the total leukocyte count increased from 8 ~ 24 hours after oral administration of the drug dosage form of this invention, reached a peak at 48 hours post-dosing and returned to the initial level by 72 hours post-dosing. In dogs given a preparation not containing G-CSF (controls), on the other hand, no such pharmacologic effect was observed.

Experiment 3

Male rats (SD strain, 400 ~ 500 g body weight) were inserted cannula from stomach into duodenum with operation and were placed in separate cages for several days. After the effect of operation was not recognized, test solutions prepared in Example 8 were administered into duodenum of rats through the cannula in a dose of 15 mg/kg of G-CSF.

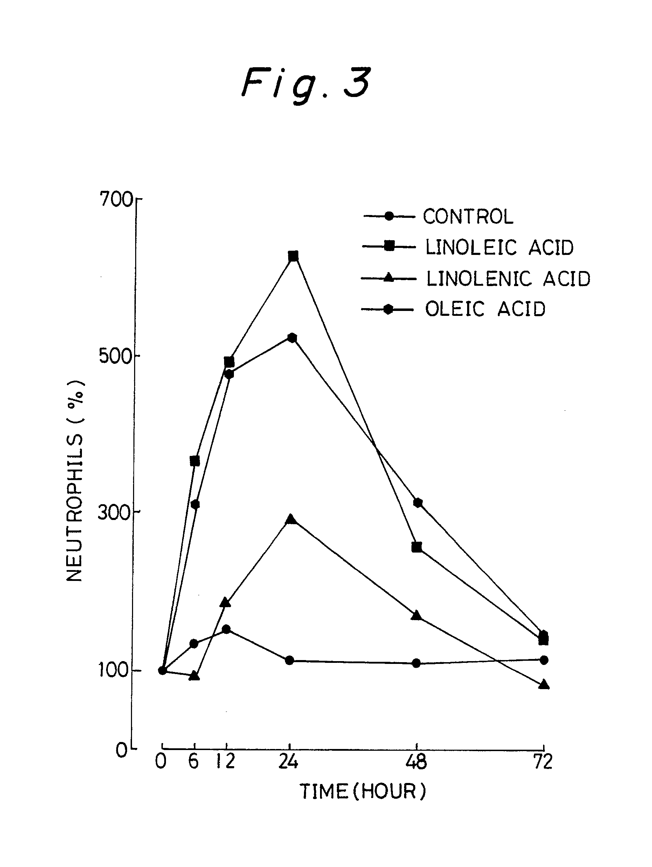

0.3 ml blood samples were taken serially following the administration from the jugular vein of each rat. Each blood sample was collected in a polystyrene tube treated with EDTA and one part of each sample was subjected to determination of blood total leukocyte count on an automated blood cell counter (Microcell Counter Sysmex CC-180A) and another part was used for determination of a ratio of neutrophil against total leukocyte from observing smears under microscope. Smears were prepared with spreading blood samples on slide glasses and subsequent staining. Then, neutrophil count in blood was calculated from total leukocyte count and neutrophil ratio. Increasing ratio of Neutrophil count at each sampling point against the initial value was showed in the Figure 3.

As seen, remarkable increase of neutrophil count was observed from 6 hr to 48 hr after administration of a solution containing fatty acid prepared according to this invention. In contrast, only slight increase was seen at 6 hr and 12 hr after dosing of preparation without fatty acid (control). Among fatty acids, preparations containing oleic acid or linoleic acid showed large pharmacological effect.

Experiment 4

Male rats (SD strain, 400 ~ 500 g body weight) were inserted cannula from stomach into duodenum with operation and were placed in separate cages for several days. After the effect of operation was not recognized, test solutions prepared in Example 9 were administered into duodenum of rats through the cannula in a dose of 2.4 mg/kg of G-CSF.

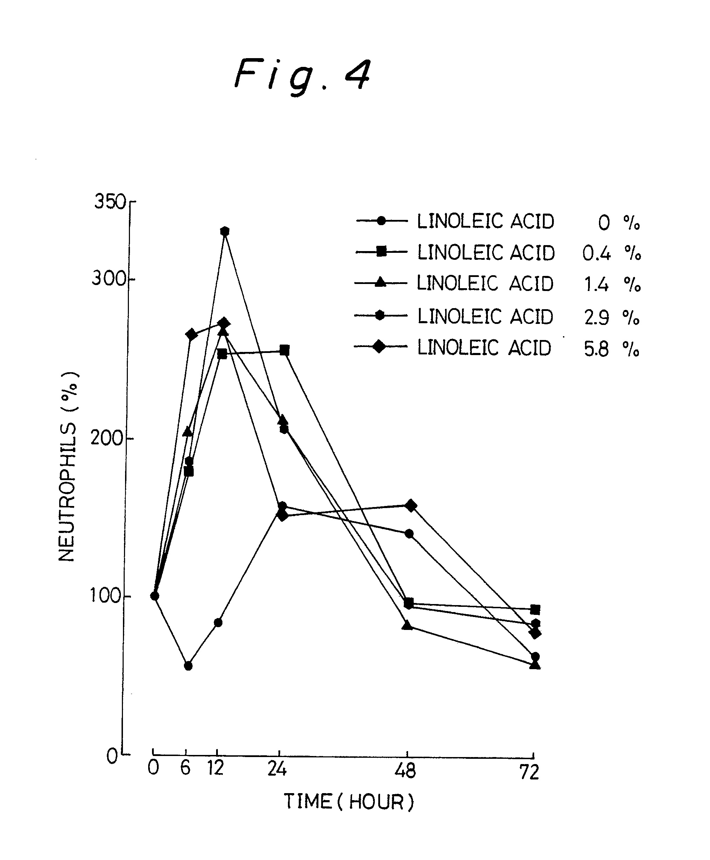

0.3 ml blood samples were taken serially following the administration from the jugular vein of each rat. Each blood sample was collected in a polystyrene tube treated with EDTA and one part of each sample was subjected to determination of blood total leukocyte count on an automated blood cell counter (Microcell Counter Sysmex CC-180A) and another part was used for determination of a ratio of neutrophil against total leukocyte from observing smears under microscope. Smears were prepared with spreading blood samples on slide glasses and subsequent staining. Then, neutrophil count in blood was calculated from total leukocyte count and neutrophil ratio. Increasing ratio of neutrophil count at each sampling point against the initial value was showed in the Figure 4.

As seen, remarkable increase of neutrophil count was seen at 6 hr and 12 hr after administration of each preparation containing 0.4%, 1.4%, 2.9% or 5.8% of linoleic acid respectively produced by this invention. On the other hand, such pharmacological effect was almost never observed after administration of the preparation without linoleic acid. In this experiment, any significant difference was not found in the addition of 0.4 to 5.8% of linolic acid (in a ratio between 1.9 and 45 parts by weight to one part by weight of G-CSF).

With the oral drug preparations provided by the present invention, therefore, inactivation of the principal ingredient during the process of pharmaceutical manufacturing has been shown to be avoided along with proven effects such as enhanced absorption of the principal ingredient from the intestinal tract particularly as a result of the addition of fatty acid to the drug composition.

These may be said not only to lead to a dosage reduction but also to facilitate accurate dose control and increase the practical usefulness of the bioactive proteins in the form of oral preparations.

The features disclosed in the foregoing description, in the claims and/or in the accompanying drawings may, both, separately and in any combination thereof, be material for realising the invention in diverse forms thereof.

## Claims

1. An oral dosage form comprising
   (a) granulocyte colony-stimulating factor or erythropoietin,
   (b) surfactant(s),
   (c) fatty acid(s), and
   (d) enteric material.

# *Fig. I*

# Fig. 2

# *Fig. 3*

# Fig. 4

European
Patent Office

**EUROPEAN SEARCH
REPORT**

Application Number

**EP 91 10 8963**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | WO-A-9 001 329 (TAKADA K.)<br>* pages 6 - 7 * | 1 | A 61 K 37/02<br>A 61 K 9/48 |
| A | EP-A-0 225 189 (R.P. SCHERER CORPORATION)<br>* claims 14-19 * | 1 | |
| A,D | EP-A-0 178 665 (CHUGAI)<br>* abstract & JP-A-61097229 * | 1 | |
| A,D | EP-A-0 178 576 (CHUGAI)<br>* abstract & JP-A-61091131 * | 1 | |
| A,D | GB-A-2 177 914 (CHUGAI)<br>* abstract & JP-A-62089627 * | 1 | |
| A,D,D,D-,D | DE-A-3 723 781 (CHUGAI)<br>* abstract & JP-A-63146826 & JP-A-63146827 & JP-A-63146828 & JP-A-63152326 * | 1 | |
| A,D | EP-A-0 263 490 (CHUGAI)<br>* abstract & JP-A-63091325 * | 1 | |

TECHNICAL FIELDS
SEARCHED (Int. Cl.5)

A 61 K
C 07 K

The present search report has been drawn up for all claims

| Place of search | Date of completion of search | Examiner |
|---|---|---|
| Berlin | 10 September 91 | AVEDIKIAN P.F. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

&: member of the same patent family, corresponding
document